# EUROPEAN PATENT APPLICATION

(11) **EP 1 992 344 A1**
(43) Date of publication of application: **19.11.2008**
(21) Application number: 07301049.8
(22) Date of filing: 18.05.2007
(51) Int. Cl.: A61K 31/404, A61K 31/4439, A61P 35/00, A61P 17/00

(54) **P38 alpha as a therapeutic target in pathologies linked to FGFR3 mutation**

(71) Applicant: Institut Curie, 75248 Paris Cedex 05 (FR); Centre National de la Recherche Scientifique, 75016 Paris (FR); Assistance Publique Hopitaux De Paris, Carré Historique de l'Hôpital Saint-Louis 1 avenue Claude Vellefaux 75010 Paris (FR); Universite Paris 12, 94010 Creteil (FR)
(72) Inventor: Bernard-Pierrot, Isabelle, 94130, Nogent Sur Marne (FR); Radvanyi, François, 92260, Fontenay Aux Roses (FR); Allory, Yves, 75011, Paris (FR)
(74) Representative: Gallois, Valérie

(57) **Abstract**

The present invention concerns methods and compositions for treating pathologies linked to FGFR3 mutations by p38 inhibitors.

## Description

### Field of the Invention

The present invention relates to the field of medicine, in particular to compositions and methods for the treatment of pathologies linked to FGFR3 mutation.

### Background of the Invention

FGFR3 (fibroblast growth factor receptor 3) belongs to a family of structurally related tyrosine kinase receptors (FGFR1-4) involved in many aspects of embryogenesis and tissue homeostasis. These receptors regulate various biological processes, including proliferation, differentiation, migration and apoptosis. They are also involved in pathological conditions and many studies have stressed their importance in developmental genetic diseases and benign or malignant tumours. FGFRs have an extracellular ligand-binding domain composed of two or three immunoglobulin-like domains, a transmembrane region and a cytoplasmic domain with tyrosine kinase activity. Ligand binding to the extracellular domain leads to FGFR dimerisation, inducing receptor activation by transphosphorylation of intracellular tyrosines. The intracellular domain then interacts with, and phosphorylates various intracellular signalling proteins. Alternative mRNA splicings affect different regions of the FGFRs. The second part of the juxtamembrane Ig-like domain of FGFR1-3 is encoded by two mutually exclusive exons resulting in two different isoforms, b and c, having different ligand specificity and tissue distribution. FGFR3b is mainly expressed in epithelial cells whereas FGFR3c is predominantly found in cells derived from the mesenchyme, including chondrocytes.

Specific germline point mutations in FGFR3 are associated with various autosomal dominant human skeletal disorders. All these mutations generate receptors auto-activated by various mechanisms, depending on the position and nature of the nucleotide substitution.

The same activating mutations of FGFR3 have been identified in benign and malignant tumours. Benign tumours include tumours in the skin (seborrheic keratosis and epidermal nevi) and in the bladder (bladder papilloma). Malignant tumours include a haematological cancer, multiple myeloma, and carcinomas of the bladder and cervix. The frequency of *FGFR3* mutations is low in multiple myelomas and cervix carcinomas (less than 2% of tumours present these mutations), but much higher in bladder carcinomas (around 50%). The transforming properties of mutated FGFR3c, the main FGFR3 isoform expressed in multiple myeloma, have been clearly demonstrated. Despite the fact that the mutated FGFR3b isoform is found mainly in benign tumours or in tumours of low malignant potential, the inventors recently clearly demonstrated the oncogenic properties of mutated FGFR3b in NIH-3T3 cells and in a bladder carcinoma cell line. Indeed, FGFR3b-S249C, the most common mutated form of FGFR3 in bladder tumours, was able to transform NIH-3T3 cells, inducing their anchorage-independent growth and tumour formation when injected subcutaneously into nude mice and FGFR3b-Y375C knock-down in MGH-U3 cells using siRNA decreased cell growth and inhibited anchorage-independent growth (Bernard-Pierrot, 2006, Carcinogenesis 4:740-747). Nevertheless, the molecular mechanisms associated to (responsible for) these oncogenic properties of mutated FGFR3 receptors were not elucidated.

Bladder cancer is the fourth cancer in men in term of incidence and the ninth in women. Per patient, it is the most costly cancer for the society. However, the number of available treatments of pathologies linked to FGFR3 mutations is very limited and new treatments are needed.

An abstract of AACR in April 2005 from the inventors discloses an *in vitro* result relating to a transfected NIH-3T3 laboratory cell line by a mutated FGFR3-S249C. FGFR3-S249C activates ERK1/2 and the P13Kinase and it specifically activates p38 MAPKinase pathway. Treatment of the transfected cell line by LY294002 or SB203580, inhibitors of P13Kinase and p38 MAPKinase, respectively, inhibits the transformed phenotype of the cells and their abilities to form colonies in soft agar. Accordingly, the authors concluded that both P13Kinase and p38 pathways are required to achieve FGFR3-S249C-induced cell transformation. The goal of the authors was the discovery of the oncogenesis mechanisms. There is no suggestion to use one of these inhibitors for any treatment. In addition, these data concerns only *in vitro* results on a laboratory model cell-line and the man skilled in the art knows that it is impossible to deduce any possible therapeutic efficiency from such *in vitro* results. Finally, there is no information about the isoform of the p38.

### Summary of the Invention

The inventors established that p38α is a therapeutic target for pathologies linked to FGFR3 mutations, in particular for the following reasons:
- p38α isoform is the only isoform involved in proliferation induced by mutated FGFR3.
- p38α inhibitors seem to be specific of mutated FGFR3. Indeed, they do not block the proliferation induced by activated wild-type FGFR3 in 3T3 cells
- p38α inhibitors block proliferation and ability to form colonies in soft agar induced by mutated FGFR3 in a bladder tumor cell line.
- p38α inhibitors block proliferation *in vivo,* in particular they reduce bladder tumor xenograft growth in nude mouse and also tumor cell proliferation in a skin mouse tumor model.
- p38α inhibitors are efficient for several FGFR3 mutations, in particular S249C and Y375C.

Therefore, p38α inhibitors are an attractive treatment against pathologies linked to FGFR3 mutations due to their targeted effect on affected cells. Accordingly, the present invention concerns the use of a p38α inhibitor for preparing a medicament for treating a pathology linked to FGFR3 mutations. The present invention also concerns a method for treating a subject suffering of a pathology linked to FGFR3 mutations comprising administering a therapeutically efficient amount of a p38α inhibitor.

In a particular embodiment, the p38α inhibitor is selected in the group consisting of AMG 548, ARQ 101, ARRY-371797, ARRY-614, AR00182263, AZD6703, RPR200765A, RPR203494, BIRB796, SB242235, SB239063, SB681323, R04402257, R03201195, RWJ67657, RWJ67671, RWJ67568, RWJ67411, RWJ66430, KC706, L-167307, SC-80036, SCIO-469, SCIO-323, SD-282, SCI-496, TAK715, VX-702, VX-850, VX-745, SB202190, SB203580, SB220025, SB239063, SC68376, Compound 37 (Amgen's 657417), SX-011, and SKF-86002. In a preferred embodiment, the p38α inhibitor is specific of p38α. For instance, the specific p38α inhibitor can be selected in the group consisting of AMG 548, ARRY-371797, ARRY-614, AR00182263, Compound 37 (Amgen's 657417), SX-011, SB203580, BIRB796, VX-745, SCIO-469 and SD-282.

In another particular embodiment, the p38α inhibitor is a siRNA directed against p38α.

In another particular embodiment, the p38α inhibitor is an antisense, a ribozyme or an antibody.

The pathology linked to FGFR3 mutations can be selected from the group consisting of multiple myeloma, benign epithelial tumours, carcinomas and congenital chondrodysplasias. Preferably, the pathology is selected from the group consisting of bladder carcinoma, achondrodysplasia and benign skin cancers, preferably carcinoma and benign skin cancers (e.g., a seborrheic keratosis or an epidermal nevus).

In a preferred embodiment, the subject to be treated has a mutated FGFR3, preferably in the tissue affected by the pathology.

In a particular embodiment, the FGFR3 mutation is selected from the group consisting of R248C, S249C, G372C, S373C, Y375C, K652E, K652M, J809G, J809C, J809R, J809L, P250R, G377C, G382R, A393E, and N542K, preferably R248C, S249C, G372C, K652E and Y375C, more preferably S249C or Y375C (the codon numbering corresponds to the FGFR3b isoform).

In a preferred embodiment, p38α inhibitor is administered orally, topically, or parentally.

The present invention also concerns a method for selecting a subject suitable to be treated by a p38α inhibitor comprising determining in a sample from a subject if FGFR3 has a mutation, and selecting the subject having a mutated FGFR3.

The present invention further concerns methods for identifying/screening a compound suitable for treating a pathology linked to FGFR3 mutations.

### Description of the Figures

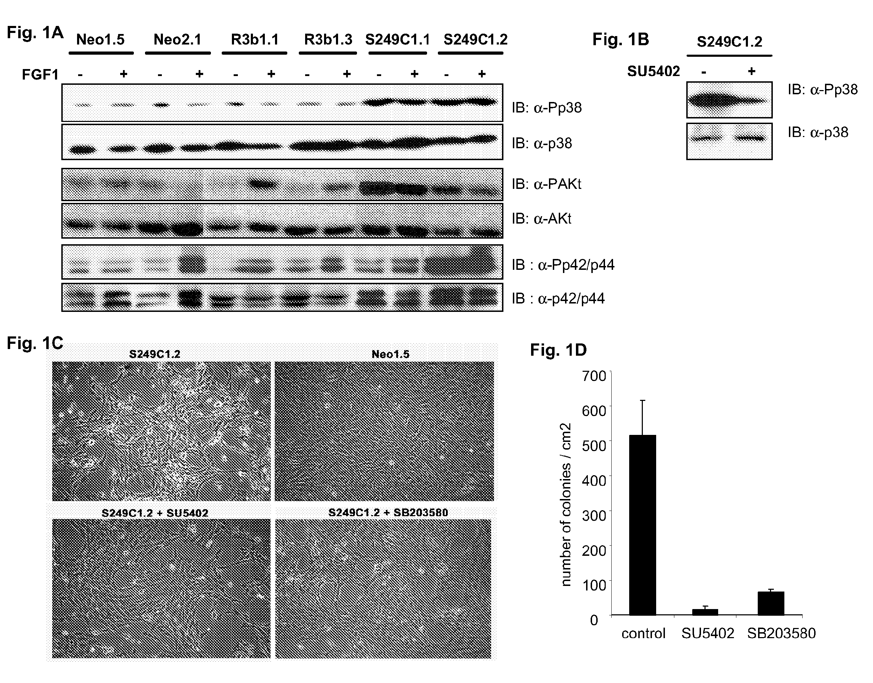

**Figure1****: FGFR3b-S249C-induced NIH-3T3 cell transformation is p38-dependent.** Fis. 1A) NIH-3T3 control cells (Neo1.5, Neo2.3), FGFR3-S249C-expressing cells (S249C1.1, S249C1.2) and wild-type FGFR3-expressing cells (R3b1.1, R3b1.3) were serum starved for 24h and stimulated, where indicated, with 20 ng/ml FGF1 in the presence of 50µg/ml heparin. 75 µg whole cell lysate proteins were immunoblotted using the following specific antibodies: anti-phospho-p38 (Pp38), anti-p38, anti-phospho-Akt (PAkt), anti-Akt, anti-phospho-p42/p44 (ERK1/2) (Pp42/p44) and anti-p42/p44 antibodies. Fig. 1B) Cell lysates (75 µg of protein) of S249C1.2 cells treated for 24 hours with 30 µM SU5402 (FGFR inhibitor) and untreated cells were immunoblotted with anti-phospho-p38 (Pp38) and anti-p38 antibodies. Fig. 1C) Morphological analysis of NIH-3T3 control cells (neo1.5) and NIH-3T3 cells expressing FGFR3-S249C (clone S249C1.2) treated for 48 hours with 30 µM SU5402 or 20 µM SB203580 (p38 inhibitor). Fig. 1D) Soft agar assay of FGFR3-S249C-expressing cells (S249C1.2) in the presence or absence of 30 µM SU5402 or 20 µM SB203580. After 21 days, colonies with diameters greater than 50 µm were counted. Data are the means of two independent experiments carried out in triplicate and the standard errors are indicated.

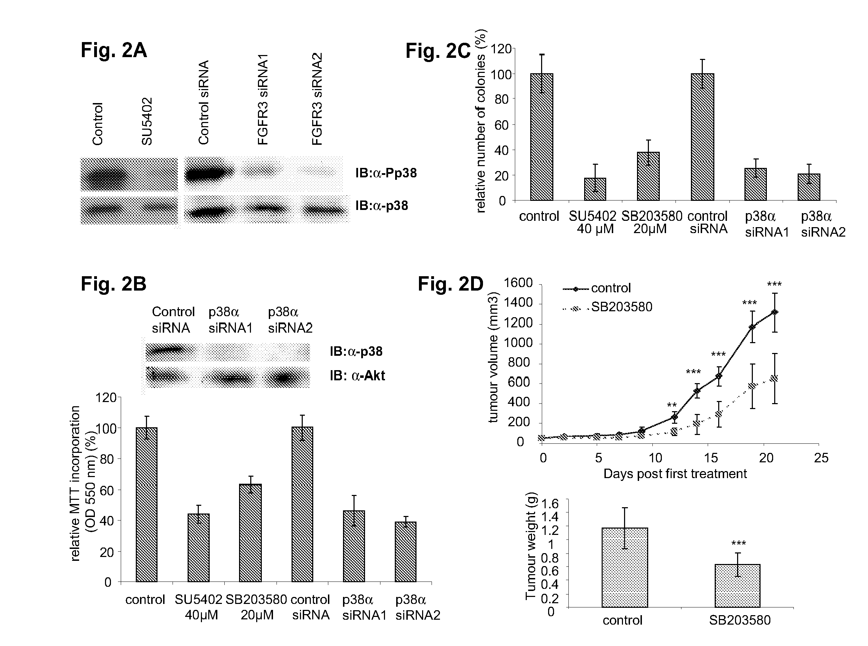

**Figure 2****: p38 depletion or inhibition inhibits MGH-U3 cell proliferation and transformation *in vitro* and *in vivo.*** Fig. 2A) FGFR3 inhibition or depletion inhibits p38 activation in MGH-U3 cells. Cell lysates (75 µg of protein) of MGH-U3 cells treated for 24 hours with 30 µM SU5402 (FGFR inhibitor), untreated cells and siRNA transfected cells (72 h post- transfection) were immunoblotted with anti-phospho-p38 (Pp38) and anti-p38 antibodies. Fig. 2B) Inset, cell lysates (50µg) of siRNA transfected MGH-U3 cells (72 h post- transfection) were immunoblotted with anti-p38 antibodies or anti-Akt antibodies. p38 inhibition or depletion inhibited MGH-U3 cell proliferation. MTT incorporation was evaluated 72 hours after treatment with SB203580 or transfection with 200 nM p38α siRNA. Fig. 2C) p38 inhibition using SB203580, or p38α depletion using siRNA, inhibited the anchorage-independent growth of MGH-U3 cells. Results are the means of two independent experiments carried out in triplicate and the standard errors are indicated. Fig. 2D) p38 inhibition inhibits MGH-U3 xenograft growth. Mice (n=5 per groups) were treated daily intratumorally with SB203580 or PBS. Day O corresponds to 6 days after inoculation of cells when tumour volume was about 50 mm³. Tumour diameters were measured at regular interval with callipers and tumour volume was calculated. At the end of the experiment tumours were weighted. Results are the means +/- SE (n=10 tumours). Statistical analyses were performed using Wilcoxon unpaired test. *, p<0.05; ** p<0.01, *** p<0.001.

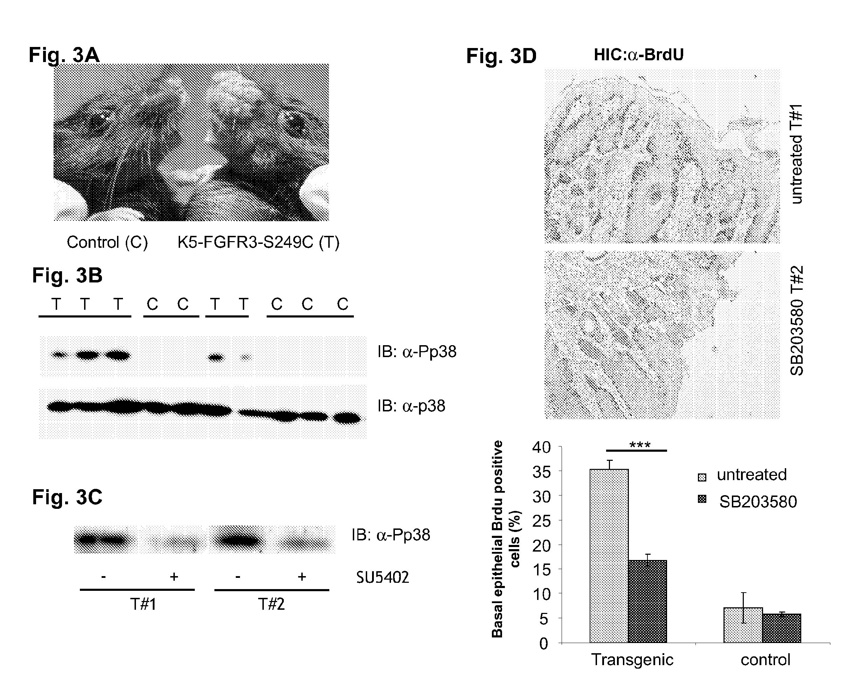

**Figure 3** **: p38 activation by FGFR3b-S249C is required to induce snout tumour epidermal cell proliferation in K5-FGFR3-S249C transgenic mice.** Fig. 3A) Phenotypes of K5-FGFR3-S249C transgenic mice (T) and littermate control mice (C). Fig. 3B) Immunoblot analysis of 75 µg snout total lysates using phospo-p38 (Pp38) and p38 antibodies. T, transgenic mouse, C, littermate control mouse. Fig. 3C) Snout of transgenic mice (T) were treated daily during 2 weeks with acetone or 100 µM SU5402. 75 µg snout total lysates were immunoblotted using phospo-p38 (Pp38). Fig. 3D) Snout of transgenic mice (T) or littermate control mice (C) were treated daily with acetone or 100 µM SB203580 during 2 weeks. Basal epidermal cells proliferation was assessed by BrdU labelling. Typical labelling for transgenic mice snout treated with SB203580 or untreated are pictured, magnification x100. Results are the means +/- SD (n=5 mice). Statistical analysis were performed using unpaired student t test, *** p<0.001.

### Detailed description of the Invention

### Definitions

Where "comprising" is used, this can preferably be replaced by "consisting essentially of", more preferably by "consisting of".

Whenever within this whole specification "treatment of a pathology linked to FGFR3 mutation" or the like is mentioned with reference to a p38α inhibitor, there is meant:
a) a method of treatment (= for treating) of a pathology linked to FGFR3 mutation, said method comprising the step of administering (for at least one treatment) a p38α inhibitor, (preferably in a pharmaceutically acceptable carrier material) to a subject, especially a human, in need of such treatment, in a dose that allows for the treatment of said pathology (= a therapeutically effective amount);
b) the use of a p38α inhibitor for the treatment of a pathology linked to FGFR3 mutation; or a p38α inhibitor for use in said treatment (especially in a human);
c) the use of a p38α inhibitor for the manufacture of a pharmaceutical preparation for the treatment of a pathology linked to FGFR3 mutation;
d) a pharmaceutical preparation comprising a dose of a p38α inhibitor that is appropriate for the treatment of a pathology linked to FGFR3 mutation; and/or
e) a product containing a p38α inhibitor and an other drug as a combined preparation for simultaneous, separate or sequential use in the treatment of a pathology linked to FGFR3 mutation.

Therefore, the present invention concerns the use of a p38α inhibitor for preparing a medicament for treating a pathology linked to FGFR3 mutations. The present invention also concerns a method for treating a subject suffering of a pathology linked to FGFR3 mutations comprising administering a therapeutically efficient amount of a p38α inhibitor. In a preferred embodiment, the p38α inhibitor is specific of p38α. p38α inhibitor is preferably an inhibitor as detailed below.

In a preferred embodiment, the pathology is selected from the group consisting of multiple myeloma, carcinomas (malignant epithelial tumours), benign epithelial tumours and congenital chondrodysplasias. In a particular embodiment, the epithelial tumour is selected from the group consisting of bladder, cervix, lung, breast, colon and skin tumours, preferably bladder carcinoma and benign skin tumours. In a preferred embodiment, the benign skin tumor is a seborrheic keratosis or an epidermal nevus. In a particular embodiment, the congenital chondrodysplasia is selected from the group consisting of hypochondrodysplasia, achondroplasia, severe achondroplasia with developmental delay and acanthosis nigricans, and thanatophoric dysplasia. In a preferred embodiment, the congenital chondrodysplasia is achondroplasia. In a more preferred embodiment, the pathology is a bladder carcinoma.

In a preferred embodiment, the subject to be treated suffering of a pathology linked to FGFR3 mutations has a FGFR3 mutation, in particular an activating FGFR3 mutation. In a particular embodiment, the FGFR3 mutation is detected in a tissue affected by the pathology. The FGFR3 mutations are further detailed below. Alternatively, the FGFR3 mutation can be detected indirectly, in particular through p38α activation, for instance by detecting phosphorylated p38α.

Accordingly, the present invention concerns a method for treating a subject suffering of a disease that can be linked to FGFR3 mutations comprising: determining in a sample from the subject if FGFR3 has a mutation, and administering a therapeutically efficient amount of a p38α inhibitor to the subject having a mutated FGFR3. Alternatively, the present invention concerns a method for treating a subject suffering of a disease that can be linked to FGFR3 mutations comprising: determining in a sample from the subject if p38α is activated, and administering a therapeutically efficient amount of a p38α inhibitor to the subject having an activated p38α. Activated p38α is a phosphorylated p38α. It can be detected with an antibody specific against the phosphorylated p38α, for instance as detailed in the examples.

The present invention further concerns the use of a p38α inhibitor for preparing a medicament for treating a pathology linked to FGFR3 mutations in a subject having a mutated FGFR3. In a particular embodiment, the present invention concerns the use of a p38α inhibitor for preparing a medicament for treating a bladder carcinoma in a subject having a mutated FGFR3. It also concerns the use of a p38α inhibitor for preparing a medicament for treating a benign skin cancer in a subject having a mutated FGFR3.

The present invention also concerns a method for selecting a subject suitable to be treated by a p38α inhibitor comprising: determining in a sample from a subject if FGFR3 has a mutation, and selecting the subject having a mutated FGFR3.

In a preferred embodiment of the methods and uses of the present invention, the mutation of FGFR3 is an activating mutation.

In a preferred embodiment, the disease is selected from the group consisting of multiple myeloma, carcinomas (malignant epithelial tumours), benign epithelial tumours and congenital chondrodysplasias. In a particular embodiment, the epithelial tumour is selected from the group consisting of bladder, cervix, lung, breast, colon and skin tumours, preferably bladder carcinoma and benign skin tumours. In a preferred embodiment, the benign skin tumor is a seborrheic keratosis or an epidermal nevus. In a particular embodiment, the congenital chondrodysplasia is selected from the group consisting of hypochondrodysplasia, achondroplasia, severe achondroplasia with developmental delay and acanthosis nigricans, and thanatophoric dysplasia. In a preferred embodiment, the congenital chondrodysplasia is achondroplasia. In a more preferred embodiment, the disease is a bladder carcinoma.

The step of determining the presence of a FGFR3 mutation is performed by any method well-known by the man skilled in the art. FGFR3 mutation can be detected at the nucleic acid level with appropriate probe(s) and/or primer(s) or at the polypeptide level with an appropriate antibody. For instance, such a method is described in the patent application WO 00/68424, the disclosure of which is incorporated herein by reference.

The sample can be selected from a group consisting of a tissue, bone marrow and a fluid such as blood and urine. In case of a skin tumour, the sample is preferably a skin biopsy or a blood sample. In case of a bladder carcinoma, the sample is preferably a bladder tumour biopsy, a blood sample or a urine sample. In case of an achondrodysplasia, the sample is a blood sample.

The subject can be an animal, preferably a mammal, more preferably a human.

The present invention further concerns a method for identifying/screening a compound suitable for treating a pathology linked to FGFR3 mutations comprising contacting a cell expressing a mutated FGFR3 with a candidate compound, and selecting the candidate compound that reduces or inhibits the proliferation of the cell and/or the ability to form colonies in soft agar.

The present invention also concerns a method for identifying/screening a compound suitable for treating a pathology linked to FGFR3 mutations comprising administering a candidate compound to an athymic nude mouse having a xenograft tumor with cells expressing a mutated FGFR3, sizing the xenograft tumor after a period of time, and selecting the candidate compound that reduces or blocks the tumor growth.

The present invention further concerns a method for identifying/screening a compound suitable for treating a pathology linked to FGFR3 mutations comprising administering a candidate compound to a transgenic non-human animal expressing a mutated FGFR3, and selecting the candidate compound that reduces or inhibits the proliferation of the tumor cell.

### p38 inhibitors

Mitogen-activated protein kinase p38 is a serine/threonine kinase originally isolated from lipopolysacharide (LPS) stimulated monocytes. There are four isoforms, namely p38α, p38β, p38γ and p38δ. p38α may also be referred to as MAPK14 (mitogen-activated protein kinase 14), CSBP, or SAPK2A (GeneID: 1432 ; Uniprot Q16539). This isoform is a therapeutic target for inflammation. Therefore, many p38α inhibitors have been developed.

p38α inhibitors can be small molecules, siRNA (e.g., US2005/0239731; WO 04/097020; WO 03/072590), antisenses, ribozymes or antibodies.

In a preferred embodiment, p38α inhibitors are siRNA, for instance as described in the examples.

In a preferred embodiment, p38α inhibitors are small molecules. Examples of p38α inhibitors that can be used in the present invention, without being limited thereto, are selected in the group consisting of AMG 548 (Amgen); ARQ 101 (Arqule); ARRY-371797, ARRY-614, and AR00182263 (ARRAY BIO-PHARMA); AZD6703 (Astrazeneca); RPR200765A and RPR203494 (Aventis); BIRB796 (Boehringer Ingelheim); SB242235, SB239063 and SB681323 (Glaxosmithkline); R04402257 and R03201195 (Hoffman-Laroche); RWJ67657, RWJ67671, RWJ67568, RWJ67411, RWJ66430 (Johnson&Johnson Pharmaceutical); KC706 (Kemia); L-167307 (Merck); SC-80036 (Pfizer); SCIO-469, SCIO-323, SD-282 and SCI-496 (Scios); TAK715 (Takeda); VX-702, VX-850, and VX-745 (Vertex); and SB202190, SB203580, SB220025, SB239063, SC68376, SKF-86002, Compound 37 (Amgen's 657417), SX-011, P38 MAPKinase inhibitor III (ref cat N°506121) and P38 MAPKinase inhibitor (cat N°506126) (Calbiochem). Other p38α inhibitors are described in the following patents and patent applications US 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, US 5,716,955, US 7,071,198, WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO99/59959, WO 99/00357, WO 99/03837, WO 99/01441, WO99/01449, WO 99/03484, WO 99/15164, WO 99/32110, WO99/32111, WO 99/32463, WO 99/64400, WO 99/43680, WO 99/17204, WO 99/25717, WO 99/50238, WO 99/61437, WO99/61440, WO 00/26209, WO 00/18738, WO 00/17175, WO00/20402, WO 00/01688, WO 00/07980, WO 00/07991, WO00/06563, WO 00/12074, WO 00/12497, WO 00/31072, WO00/31063, WO 00/23072, WO 00/31065, WO 00/35911, WO 00/39116, WO 00/43384, WO 00/41698, WO 00/69848, WO00/26209, WO 00/63204, WO 00/07985, WO 00/59904, WO00/71535, WO 00/10563, WO 00/25791, WO 00/55152, WO00/55139, WO 00/17204, WO 00/36096, WO 00/55120, WO00/55153, WO 00/56738, WO 01/21591, WO 01/29041, WO 01/29042, WO 01/62731, WO 01/05744, WO 01/05745, WO01/05746, WO 01/05749, WO 01/05751, WO 01/27315, WO01/42189, WO 01/00208, WO 01/42241, WO 01/34605, WO01/47897, WO 01/64676, WO 01/37837, WO 01/38312, WO01/38313, WO 01/36403, WO 01/38314, WO 01/47921, WO 01/27089, WO0246158, WO03002542, WO03097062, WO03103590, WO03099796, WO03084539, WO03097062, WO03104223, WO04099156, WO04110990 WO04092144, WO04022712, WO04016267, WO04113348 WO04004725, WO04032874, WO04076450, WO04020440, WO04078116 US2004092547, WO04020438, WO04014900, WO04014920, WO04091625, WO05077945, WO05100338, WO05061486, WO05060967, WO05105743, WO05000405, WO05073232, WO05073189, WO05073217, WO05073219, WO05033102, WO05012241, WO05030151, WO05105790, WO05032551, WO05075478, WO05076990, WO05009973, WO05075425, WO05000298, WO05080380, WO05009367, WO05033086, WO05034869, WO05033072, WO05042540, WO05082862, WO05063766, WO05120509, WO05097758, WO06127678, WO06118914 WO06071456, US2006234911, WO06055302, EP1707205, WO06099495, WO06116355 WO06122230, WO06084017, WO06067168, WO06104915, WO06062982, WO06112828 WO06028836, WO06018842, WO06047354, WO06020904, WO06040056, WO06006691, WO06009741, WO06044869, WO06018727, WO06018735, WO06052810, US2006058296, WO06010082, WO06003517, WO06020365, US2006079461, WO06104889, WO06018718, WO06128268, WO06110173 WO06039718, WO06040666, WO06040649, WO06013095, US2006052390, WO07015877, WO07015866, WO07000340, WO07000339, WO07000337, WO07016392, DE 19842833, and JP 2000 86657, whose disclosures are all incorporated herein by reference in their entirety.

p38α inhibitors selective against the isoform α are preferred in the present invention. Such a p38α specificity avoids any potential side effect due to inhibition of other p38 isoforms. By "selective against" is intended herein that the inhibitor is more efficient for inhibiting p38α than at least one of other isoforms of p38, preferably the three other isoforms. More preferably, selective p38α inhibitors have almost no inhibiting effect against the other isoforms, and still more preferably no inhibiting effect at all, in particular at the therapeutically effective amount used for the treatment. In a particular embodiment, IC50 of the inhibitor is at least 5, 10, 50, 100, 500, 1000, 5000 or 10000 fold greater for the other p38 isoforms in comparison with the IC50 for p38α (IC50 being the dose necessary to have 50 % inhibition of p38α kinase activity). Examples of p38α inhibitors that can be used in the present invention, without being limited thereto, are selected in the group consisting of AMG 548 (Amgen); ARRY-371797, ARRY-614, et AR00182263 (ARRAY BIO-PHARMA); Compound 37 (Amgen's 657417) and SX-011 (Lee & Dominguez, Curr Med Chem, 2005, 12, 2979-2994); SB203580 (Calbiochem); BIRB796 (Boehringer Ingelheim); VX-745 (Vertex); and SCIO-469 and SD-282 (Scios).

The therapeutically effective amount of p38α inhibitors varies depending upon the administration mode, the age, body weight, sex and general health of the subject. It will be appreciated that there will be many ways known in the art to determine the therapeutically effective amount for a given application.

The therapeutically effective amount of p38α inhibitors is preferably the amount sufficient for inhibit the cell proliferation, preferably cancer cell proliferation, and/or for reducing or blocking the tumor growth, and/or for reducing or inhibiting the transformed phenotype, and/or for reducing or inhibiting the cell ability to form colonies in soft agar.

The appropriate dose can be from 0.01 to 1000 mg/day. For instance, the dose of AMG 548 can be from 0.1 mg to 1000 mg/day, preferably between 1 and 500 mg/day by oral administration. The dose of BIRB796 can be from 0.1 mg to 500 mg/day, preferably between 10 and 200 mg/day by oral administration.

The therapeutically effective amount of p38α inhibitors can be administered once, twice, thrice, or four times a day. Alternatively, the therapeutically effective amount of p38α inhibitors can be administered every day, every two day, or one, two, or three times a week.

The p38α inhibitor may be administered orally, parenterally, by inhalation spray, topically, rectally, nasally, buccally, vaginally or via an implanted reservoir. The term "parenteral" as used herein includes subcutaneous, intravenous, intramuscular, intra-tumoral, intra-articular, intra-synovial, intrasternal, intrathecal, intrahepatic, and intralesional. Preferably, the p38α inhibitor is administered orally, intraperitoneally, intra-tumoral, intra-articular, intra-synovial, topically, or intravenously.

p38α inhibitor used in the present invention can be formulated as a pharmaceutical composition generally comprising a pharmaceutically acceptable carrier. By a pharmaceutically acceptable carrier is intended a carrier that is physiologically acceptable to the treated mammal while retaining the therapeutic properties of the drug with which it is administered. For example, a pharmaceutically acceptable carrier can be physiological saline solution. Other pharmaceutically acceptable carriers are known to one skilled in the art and described for instance in Remington: The Science and Practice of Pharmacy (20th ed., ed. A.R. Gennaro AR., 2000, Lippincott Williams & Wilkins).

For the benign skin cancers, a topical administration is preferred. For application topically to the skin, p38α inhibitor will be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, p38α inhibitor can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. Topically-transdermal patches and iontophoretic administration are also included in this invention.

p38α inhibitor suitable for oral administration may be presented as discrete units such as capsules, sachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion, or packed in liposomes and as a bolus, etc. Soft gelatin capsules can be useful for containing such suspensions, which may beneficially increase the rate of compound absorption.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

### Pathologies linked to FGFR3 mutations

FGFR3 refers to the fibroblast growth factor receptor 3. In particular, FGFR3 is the FGFR3b isoform in carcinomas and benign skin tumors whereas it is the FGFR3c preferred isoform in skeletal pathologies. The nucleotidic sequence of the FGFR3-IIIb isoform is described in the European patent 1 208 231 B1. The nucleotidic sequence of the FGFR3-IIIc isoform is the sequence referred in Genebank as NM_000142.

FGFR3 mutation is preferably an activating mutation. By "activating mutation" is intended herein an activating function of a mutation, which can be determined by observation of activating signals such as receptor phosphorylation or indirect effects as calcium influx, phosphorylation of target sequences. In a particular embodiment, the target sequence is p38α.

Activating FGFR3 mutations are generally in exon 7, encoding the junction between immunoglobulin-like domains II and III of FGFR3, in exon 10, encoding the transmembrane domain, in exon 15, encoding the tyrosine kinase domain I, and/or in the exon encoding the C-terminal part. Preferably, said mutations are selected in the group consisting of R248C, S249C, G372C, S373C, Y375C, K652E, K652M, J809G, J809C, J809R, J809L, P250R, G377C, G382R, A393E, and N542K. Codon and mutated nucleotide (nt position) are numbered according to the open reading frame of the FGFR3-IIIb cDNA which is the predominant form in epithelial cells. More preferably, the FGFR3 mutation is selected in the group consisting of R248C, S249C, G372C, K652E and Y375C. Still more preferably, the FGFR3 mutation is S249C or Y375C.

### Examples

The following examples will serve to further illustrate the present invention without, at the same time, however, constituting any limitation thereof. On the contrary, it is to be clearly understood that resort may be added to various other embodiments, modifications, and equivalents thereof which, after reading the description herein, may suggest themselves to those skilled in the art without departing from the spirit of the present invention and/or the scope of the appended claims.

### Materials & Methods

### Cell culture and transfection

Transfected NIH-3T3 cells expressing the wild-type FGFR3b isoform (clones R3b1.1, R3b1.3) or expressing the mutated FGFR3b-S249C receptor (clones S249C1.1, S249C 1.2) or those transfected with the control pcDNAI-Neo plasmid (clones Neo1.5, Neo 2.1) were established previously {Bernard-Pierrot, 2006, Carcinogenesis 4, 740-747 } and cultured in DMEM with 10% newborn calf serum, 2 mM glutamine, 100 u/ml penicillin, 100 µg/ml streptomycin, 400 µg/ml G418. The human bladder carcinoma cell line MGH-U3 {Lin, 1985 #96} was cultured in DMEM/F12 with 10% foetal calf serum (FCS), 2 mM glutamine. All cells were cultured at 37°C under an atmosphere containing 5% CO₂.

For siRNA transfection, MGH-U3 cells were seeded at a density of 8000 cells/cm² in 6-well or 24-well plates and transfected with 200 nM siRNA, using Oligofectamine^{®} reagent (Invitrogen, Cergy Pontoise, France) according to the manufacturer's protocol. FGFR3 siRNAs, P38α (MAPK14) siRNAs and nonsense siRNA used as a control were chemically synthesised (Ambion, Huntingdon, United Kingdom). Concerning P38α siRNA, the inventors used silencer^{®} validated siRNA (Ambion): P38α siRNA1 (siRNA ID: 1312) and P38α siRNA2 (siRNA ID: 1217). For the other siRNA the sequences of the sense strands were: control siRNA, 5'-GGCAAGAUUCUUCUCGUUGTT-3' (SEQ ID No 1); FGFR3 siRNA1, 5'-GCCUUUACCUUUUAUGCAATT-3' (SEQ ID No 2); FGFR3 siRNA2, 5'-GGGAAGCCGUGAAUUCAGUTT-3' (SEQ ID No 3). The inventors had previously shown that both FGFR3 siRNA lead to 80-90 % depletion of FGFR3 protein in MGH-U3 cells {Bernard-Pierrot, 2005}. They measured MTT (3-(4,5-dimethylthiazol-2-yl)-2,5-diphenyltetrazolium bromide) incorporation 72 h after transfection (24-well plates) to assess cell proliferation/viability or lysed them with lysis buffer (6-well plates).

### Morphology analysis

NIH-3T3 cells were cultured 48 h in presence of different inhibitors, SB203580 and SU5402 (Calbiochem, Merck Eurolab, Fontenay Sous Bois, France), and pictures were taken using a phase-contrast microscope.

### Immunoblot analysis

Transfected NIH-3T3 cells were washed once with PBS and serum-starved for 24h. Where required, cells were stimulated for 5 min with 20 ng/ml FGF1 in the presence of 50 µg/ml heparin at 37°C. They were then lysed in lysis buffer (50mM Tris-HCl (pH 7.4), 150mM NaCl, 1% NP-40, 0.25% sodium deoxycholate, 1mM EDTA, 1mM Na₃VO₄, 5mM NaF, 1µg/ml aprotinin, 1µg/ml leupeptin and 1µg/ml pepstatin) and the lysates were clarified by centrifugation. The protein concentrations of the supernatants were determined with the Biorad Bradford Protein Assay Kit (BioRad, Ivry sur Seine, France). Proteins from total cell lysates (75µg protein) were resolved by SDS-PAGE on 10 % polyacrylamide gels, electrotransferred to Immobilon-P membrane in 25mM Tris, pH 8.3, 200mM glycine, 10% ethanol and analyzed using antibodies against p38 (#9212), Akt (#9272), p42/p44 (#9102) and the phosphorylated forms of these proteins (phospho AKT (Thr308) #9275; phospho p42/p44 (thr202/tyr204) # 9101; phospho p38 (thr180/tyr182) (cell signaling Technology , Ozyme, Montigny le Bretonneux, France)

MGH-U3 total cell lysates (75 µg) were analyzed similarly using antibodies against p38, phospho-p38 and Akt.

### Soft agar assay

For NIH-3T3, 30,000 transfected cells were added, in triplicate, to each well of a 12-well plate containing DMEM supplemented with 10% NCS and solidified with agar. For MGH-U3, 20,000 non-transfected or siRNA-transfected cells were added, to triplicate wells of 12-well plates containing MEM supplemented with 10% FCS and agar. When necessary, both cell types were cultured in the presence or absence of different inhibitors in agar and in culture medium. The same amount of inhibitor was added to the culture medium weekly.

The plates were incubated for 21 or 14 days for NIH-3T3 and MGH-U3 cells respectively, and colonies with diameters greater than 50 µm were then scored as positive, using a phase-contrast microscope equipped with a measuring grid.

### Tumour formation in nude mice

Ten six-week-old female Swiss nu/nu mice were raised in the animal facilities of the Curie Institute in specified pathogen-free conditions. Their care and housing were in accordance with the institutional guidelines of the French National Ethics Committee *(Ministère de l'Agriculture et de la Forêt, Direction de la Santé et de la Protection Animale,* Paris, France) and were supervised by authorised investigators. Each mouse was injected subcutaneously in each flank (dorsal region) with 4 x 10⁶ cells/site. When tumours reached 50 mm³ (+/- 10), mice were randomly separated into two group of 5 mice. 100 µl of 20 µM SB203580 or equal volume of PBS were administrated daily in tumours. Tumour formation was monitored for up to 25 days and the size of tumours was measured with Vernier calipers: two perpendicular diameters were used to estimate tumour volumes by the formula *ab*²/2, where *a* is the largest and *b* the smallest diameter.

### Transgenic mice

Transgenic mice (K5-FGFR3-S249C) expressing mutated receptor FGFR3b-S249C in basal cells of the epidermis were established previously (Logié et al 2005, Hum. Mol. Genet. 14, 1153-1160). 40 µl of 100 µM SU5402 or of 100 µM SB203580 or of vehicule (acetone) were applied daily during 2 weeks on snout of 3 to 4 months old transgenic mice who had developed epidermal tumours on snout or of control littermates mice. Two hours prior sacrifice mice were injected (i.p), with 0.25 mg/g body weight Brdu (Sigma, Saint-Quentin Falavier, France). Brdu immunohistochemistry was performed on parafine embed snout tissue with BrdU in situ detection kit (BD Pharmingen, San Diego, CA, USA) according to the manufacturer's instructions. The number of BrdU-labeled cells among 500 cells from basal epidermis layer was determined for each mouse. Total snout cells lysate (75 µg) were analyzed as described above using antibodies against p38 and phospho-p38 (Pp38).

### RESULTS

### FGFR3b-S249C-induced NIH-3T3 cell transformation is p38 MAP Kinase activation-dependent

The inventors have previously shown that contrary to mutated FGFR3b, wild-type FGFR3b even activated by one of its ligand did not transform NIH-3T3 cells despite that the level of phosphorylation of both receptors was similar {Bernard-Pierrot, 2005} . The inventors hypothesized that the two receptors (mutated and ligand-stimulated) may be able to activate different transduction pathways accounting for the observed differences in transforming activity.

The inventors first examined the activation of three signalization pathways in NIH-3T3 cells expressing the mutant FGFR3-S249C (S249C1.1 and S249C1.2) and in FGF1-stimulated clones expressing the wild-type receptor (R3b1.1 and R3b1.3) (Figure 1A).

Both clones expressing the mutant FGFR3-S249C (S249C1.1 and S249C1.2) and FGF1-stimulated clones expressing the wild-type receptor (R3b1.1 and R3b1.3) were able to phosporylate Akt and ERK1/2 (p42/p44) but it is noteworthy that the mutated receptor induced a permanent activation of these pathways whereas the stimulated receptor induced a transient activation of these pathways (data not shown). This discrepancy could explain the observed difference in the transforming ability of the two receptors. Furthermore, interestingly, the inventors detected strong phosphorylation of p38 only in clones expressing the mutant receptor and not in FGF1-stimulated FGFR3-expressing clones (Figure 1A) (even when clones were stimulated by FGF1 during 48 hours to mimic a permanent activation of FGFR3 (data not shown)), suggesting a specific activation of p38 MAP Kinase pathway by mutated FGFR3 and the involvement of this pathway in FGFR3-S249C-induced cell transformation.

To confirm that p38 activation was due to FGFR3 activation in stably established FGFR3-S249C expressing NIH-3T3 clones, the inventors studied p38 phosphorylation in the two FGFR3-S249C expressing clones (S249C1.1 and S249C1.2) treated with SU5402, a FGFR tyrosine kinase inhibitor. As expected, p38 phosphorylation in FGFR3-S249C expressing clones was inhibited in presence of 30 µM SU5402 demonstrated that p38 activation did depend on FGFR3-S249C activation (Figure 1B).

To assess the functional significance of FGFR3-S249C-induced p38 phosphorylation in cell transformation, the evaluated the effect of SB203580, a chemical inhibitor specific for p38 MAP Kinase, on the morphology and anchorage-independent growth of clone S249C1.2. As a control, the inventors also evaluated the effect of SU5402, an inhibitor of FGFR tyrosine kinase activity. Treatment of FGFR3-S249C-expressing cells with 30µM SB203580 or with 30µM SU5402 reverted their transformed phenotype (Figure 1C) whereas both drugs had no effect on the control cell phenotype (data not shown). Both treatments also inhibited the ability of FGFR3-S249C-expressing cells to form colonies in a soft agar assay (Figure 1D). These results demonstrate that transformation of the FGFR3-S249C-expressing NIH-3T3 cells is due to FGFR tyrosine kinase activity and that it is p38-dependent.

### p38 activation by FGFR3b-Y375C is required for MGH-U3 cell proliferation and transformation

The inventors then wondered if this implication of p38 MAPKinase in mutated FGFR3-induced cell transformation was specific to NIH-3T3 cells or of more biological significance in an epithelial cell line. Since they had previously demonstrated that the tumour properties of MGH-U3, bladder carcinoma cells, depended on mutated receptor activity (FGFR3b-Y375C) {Bernard-Pierrot, 2005}, they investigated whether mutated FGFR3 induced p38 activation in this cell line.

Immunoblot analysis showed a strong phosphorylation of p38 in MGH-U3 cells (Figure 2A) and demonstrated that this p38 activation was due to FGFR3-Y375C activity since FGFR3 tyrosine kinase inhibition with SU5402 or decrease in FGFR3 expression following siRNA treatment suppressed p38 phosphorylation (Figure 2A).

To investigate the role of p38 activation by FGFR3b-Y375C, the inventors evaluated the effect of SB203580, a p38 MAP Kinase inhibitor, on MGH-U3 cell proliferation and anchorage-independent growth. As a control, they also evaluated the effect of SU5402, an inhibitor of FGFR tyrosine kinase activity. MTT incorporation demonstrated a 40 to 50 % growth inhibition in bladder cancer cells with both inhibitors (Figure 2B), and SB203580-treated cells formed one third as many colonies on soft agar as control cells (Figure 2C). However, even if SB203580 should inhibit specifically P38α (MAPK14) and p38β (MAPK11), some doubt could be raised on this specificity. Hence, to confirm the observed effects of p38 inactivation, the inventors evaluated the effect of p38α (MAPK14) depletion using siRNA on MGH-U3 cell proliferation and transformation. Indeed, since MGH-U3 cells do not express p38β (MAPK11) mRNA (data not shown), the observed effects using SB203580 were thought to be due to p38α (MAPK14) activity inhibition. Immunoblot analysis showed that 48 hours after transfection, both p38α siRNAs induced a 90 to 100 % p38α expression inhibition (Inset, Figure 2B). A 50 to 60 % growth inhibition (Figure 2B) and a 70 to 80 % anchorage independent growth (Figure 2C) of p38α siRNA-transfected cells were observed. Thus, decreases in p38α expression or activity lead to the inhibition of cell growth and cell transformation in MGH-U3 bladder cancer cells *in vitro.*

Furthermore an intratumoral treatment with SB203580 significantly reduced MGH-U3 xenograft growth in athymic nude mice, demonstrating hence the involvement of p38 activation in MGH-U3 cells transformation *in vivo* (Figure 2D).

### p38 activation by FGFR3b-S249C is required to induce basal epithelial cell hyper-proliferation in K5-FGFR3-S249C transgenic mice epidermal snout tumours.

The inventors have previously established a transgenic mouse model in which they targeted the expression of mutated FGFR3b, FGFR3b-S249C, to the basal cells of epidermis using the keratine 5 promoter {Logie, 2005, Hum. Mol. Genet. 14, 1153-1160}. These mice developed benign epidermal tumors particularly on the snout (Figure 3A). The inventors wondered so if in this model, tumour cell proliferation could also be due to p38 activation by mutated FGFR3b. First, the inventors showed by immunoblot analysis that p38 was phosphorylated in transgenic mice snout lysate whereas no phophorylation was observed in control litterates mice snout, suggesting that FGFR3-S249C expression lead to p38 activation in transgenic mice snout (Figure 3B). To validate this result, snout of transgenic mice were treated with SU5402, a FGFR tyrosine kinase inhibitor, and the phophorylation status of p38 was evaluated. Immunoblot analysis showed a decreased in p38 phosphorylation upon FGFR3 inhibition demonstrating hence that mutated FGFR3 did activate p38 pathway in transgenic mice snout cells (Figure 3C). Then, the inventors demonstrated that snout treatment with SB203580, a p38 MAP Kinase inhibitor, induced a significant decreased of transgenic mice tumor cell proliferation as assessed by BrdU labelling whereas it had no effect on control mice basal epidermal cells proliferation (Figure 3D). Taken together those results clearly pointed out the activation of P38 MAP Kinase pathway by mutated FGFR3b in basal epidermal cells and its implication in epidermal tumour cell proliferation in our skin mouse tumour model.

The inventors clearly demonstrated in three different models that mutated FGFR3 expression lead to p38 MAP Kinase activation which is required for mutated FGFR3 to induce cell transformation. Since the inventors used SB203580 which is an inhibitor more specific of p38 α and β isoforms, it can be assumes that this is one of these two p38 isoforms that is implicated in FGFR3-S249C transforming activity in NIH-3T3 cells and in transgenic mouse epidermis. The inventors demonstrated using specific siRNA that p38 α is involved in MGH-U3 cells transformation.

## Claims

1. Use of a p38α inhibitor for preparing a medicament for treating a pathology linked to FGFR3 mutations.

2. Use according to claim 1, wherein the p38α inhibitor is selected in the group consisting of AMG 548, ARQ 101, ARRY-371797, ARRY-614, AR00182263, AZD6703, RPR200765A, RPR203494, BIRB796, SB242235, SB239063, SB681323, R04402257, R03201195, RWJ67657, RWJ67671, RWJ67568, RWJ67411, RWJ66430, KC706, L-167307, SC-80036, SCIO-469, SCIO-323, SD-282, SCI-496, TAK715, VX-702, VX-850, VX-745, SB202190, SB203580, SB220025, SB239063, SC68376, Compound 37 (Amgen's 657417), SX-011, and SKF-86002.

3. Use according to claim 1 or 2, wherein the p38α inhibitor is specific of p38α.

4. Use according to claim 3, wherein the specific p38α inhibitor is selected in the group consisting of AMG 548, ARRY-371797, ARRY-614, AR00182263, Compound 37 (Amgen's 657417), SX-011, SB203580, BIRB796, VX-745, SCIO-469 and SD-282.

5. Use according to claim 1, wherein the p38α inhibitor is a siRNA directed against p38α.

6. Use according to claim 1, wherein the p38α inhibitor is selected from the group consisting of an antisens, an antibody and a ribozyme.

7. Use according to any one of claims 1-6, wherein the pathology is selected from the group consisting of multiple myeloma, carcinomas and congenital chondrodysplasias.

8. Use according to claim 7, wherein the pathology is selected from the group consisting of bladder carcinoma, achondrodysplasia and benign skin cancers, preferably carcinoma and benign skin cancers (e.g., a seborrheic keratosis or an epidermal nevus).

9. Use according to any one of claims 1-8, wherein the subject to be treated has a mutated FGFR3, preferably in the tissue affected by the pathology.

10. Use according to any one of claims 1-9, wherein the FGFR3 mutation is selected from the group consisting of R248C, S249C, G372C, S373C, Y375C, K652E, K652M, J809G, J809C, J809R, J809L, P250R, G377C, G382R, A393E, and N542K, preferably R248C, S249C, G372C, K652E and Y375C, more preferably S249C or Y375C.

11. Use according to any one of claims 1-10, wherein the p38α inhibitor is administered orally, topically, or parentally.

12. A method for selecting a subject suitable to be treated by a p38α inhibitor comprising, determining in a sample from a subject if FGFR3 has a mutation, and selecting the subject having a mutated FGFR3.

13. A method according to claim 12 wherein the FGFR3 mutation is selected from the group consisting of R248C, S249C, G372C, S373C, Y375C, K652E, K652M, J809G, J809C, J809R, J809L, P250R, G377C, G382R, A393E, and N542K, preferably R248C, S249C, G372C, K652E and Y375C, more preferably S249C or Y375C.


SEQUENCE LISTING

<110>  INSTITUT CURIE et al.

<120>  p38alpha  as a therapeutic target in pathologies linked to FGFR3
       mutation

<130>  B0564EP

<160>  3

<170>  PatentIn version 3.3

<210>  1
<211>  21
<212>  DNA
<213>  artificial sequence

<220>
<223>  siRNA

<400>  1
ggcaagauuc uucucguugt t                                                21


<210>  2
<211>  21
<212>  DNA
<213>  artificial sequence

<220>
<223>  siRNA

<400>  2
gccuuuaccu uuuaugcaat t                                                21


<210>  3
<211>  21
<212>  DNA
<213>  artificial sequence

<220>
<223>  siRNA

<400>  3
gggaagccgu gaauucagut t                                                21